# EUROPEAN PATENT SPECIFICATION

(11) **EP 4 212 158 B1**
(45) Date of publication and mention of the grant of the patent: **22.10.2025**
(21) Application number: 21865519.9
(22) Date of filing: 02.04.2021
(51) Int. Cl.: A61K 31/4406, A61P 9/04, A61K 31/00, A61K 31/21, A61K 31/455

(54) **(S)-1-((4-CYANO-3-(TRIFLUOROMETHYL)PHENYL)AMINO)-3-(4-CYANOPHENOXY)-2-METHYL-1-OXOPROPAN-2-YL NICOTINATE FOR USE IN THE TREATMENT OF HEART FAILURE**
(S)-1-((4-CYANO-3-(TRIFLUORMETHYL)PHENYL)AMINO)-3-(4-CYANOPHENOXY)-2-METHYL-1-OXOPROPAN-2-YLNICOTINAT ZUR VERWENDUNG BEI DER BEHANDLUNG VON HERZINSUFFIZIENZ
NICOTINATE DE (S)-1-((4-CYANO-3-(TRIFLUOROMÉTHYL)PHÉNYL)AMINO)-3-(4-CYANOPHÉNOXY)-2-MÉTHYL-1-OXOPROPAN-2-YL POUR UNE UTILISATION DANS LE TRAITEMENT DE L'INSUFFISANCE CARDIAQUE

(30) Priority: 14.09.2020 CN 202010959983
(43) Date of publication of application: 19.07.2023
(73) Proprietor: Changchun Genescience Pharmaceutical Co., Ltd., Changchun, Jilin 130012 (CN)
(72) Inventor: ZHU, Xinfa, Zhejiang 315102 (CN); ZHU, Ran, Zhejiang 315102 (CN)
(74) Representative: Gille Hrabal Partnerschaftsgesellschaft mbB Patentanwälte
(86) International application number: PCT/CN2021/085271
(87) International publication number: WO 2022/052456

(56) References cited:
- WO-A1-2016/044447
- WO-A2-2007/136607
- CN-A- 1 069 727
- CN-A- 101 516 835
- CN-A- 103 772 238
- CN-A- 111 138 353
- CN-A- 112 007 027

## Description

### TECHNICAL FIELD

The present disclosure belongs to the technical field of medicines, and particularly relates to (S)-1-((4-cyano-3-(trifluoromethyl)phenyl)amino)-3-(4-cyanophenoxy)-2-methyl-1-oxopropan-2-yl nicotinate for use in the treatment of heart failure.

### BACKGROUND

Due to failure of systolic and (or) diastolic functions of the heart, the blood that returns back to the heart via the veins cannot be sufficiently discharged from the heart, which results in accumulation of blood in the venous system and insufficient perfusion of blood in the arterial system, and thereby causes a heart circulatory disorder syndrome, which is called heart failure (HF, or cardiac failure). The main manifestations of this disorder syndrome include pulmonary congestion and congestion in the vena cava. The development of heart failure usually follows the process below: due to myocardial infarction, the apoptosis of cardiomyocytes occurs, followed by the progressive loss of effective contractile functional units, i.e., cardiomyocytes, enhancement of compensatory function of viable cardiomyocytes, adaptive hypertrophy of cardiomyocytes, deposition of extracellular matrix and reactive interstitial fibrosis, and reduction in the thickness of left ventricular wall. With the progression in the degree of hypertrophy, the increase in apoptosis of cardiomyocytes further reduces the number of cardiomyocytes, and the whole contractility of the myocardium is reduced. Meanwhile, after myocardial infarction, apoptotic cardiomyocytes release a large number of cytokines to promote collagen production and thereby repair injured tissues. Over-expressed collagen deposition is further manifested as myocardial fibrosis, leading to abnormal systolic and diastolic functions, and meanwhile this repair effect can be expanded to the area around the infarct area. The increase in fibrosis in turn reduces the compliance of the left ventricle, and myocardial contractility cannot exert its due ejection effect, thus forming a vicious circle and resulting in decompensation of myocardial function and ultimately heart failure.

Prior art documents WO 2016/044447 A and WO 2007/136607 A both disclose compounds for treating heart disesases.

Prior art document CN 111 138 353 A discloses the compound (S)-1-((4-cyano-3-(trifluoromethyl)phenyl)amino)-3-(4-cyanophenoxy)-2-methyl-1-oxopropan-2-yl nicotinate and its medical use against muscle diseases.

### SUMMARY

Given the above-mentioned defects of the prior art, the object of the present disclosure is to provide an ester group-containing aromatic propionamide compound for use in the treatment of heart failure, wherein the ester group-containing aromatic propionamide compound is C₂₅H₁₇F₃N₄O₄ and the chemical name thereof is (S)-1-((4-cyano-3-(trifluoromethyl)phenyl)amino)-3-(4-cyanophenoxy)-2-methyl-1-oxopropan-2-yl nicotinate.
According to one embodiment, a dose of the ester group-containing aromatic propionamide compound used in the treatment of heart failure is 1 mg/kg-10 mg/kg.

Preferably, a dose of the ester group-containing aromatic propionamide compound for the manufacturing of the medicament for the treatment of heart failure is 2 mg/kg-5 mg/kg.

Preferably, the dose of the ester group-containing aromatic propionamide compound for the manufacturing of the medicament for the treatment of heart failure is 3 mg/kg.

The present disclosure has the following outstanding effects: the ester group-containing aromatic propionamide compound can improve the situation of reduction in ejection fraction, fractional shortening and cardiac output of a chronic heart failure rat, and increase LVSP of a heart failure rat. This compound can have very positive effect in the manufacturing of a medicament for the treatment of heart failure.

The specific embodiments of the present disclosure is further described in detail with reference to the accompanying drawings of the examples, so that the technical solutions of the present disclosure can be understood and appreciated more easily.

### BRIEF DESCRIPTION OF THE DRAWINGS

FIG. 1 is a column chart showing the effect of different compounds on weight gain of rats in the present disclosure.
FIG. 2 is a column chart showing the effect of different compounds on heart weight index of rats in the present disclosure.
FIG. 3 is a column chart showing the effect of different compounds on brain/heart weight index of rats in the present disclosure.
FIG. 4 is a column chart showing the effect of different compounds on LVEF of heart failure rats in the present disclosure.
FIG. 5 is a column chart showing the effect of different compounds on FS of heart failure rats in the present disclosure.
FIG. 6 is a column chart showing the effect of different compounds on SV of heart failure rats in the present disclosure.
FIG. 7 is a column chart showing the effect of different compounds on CO of heart failure rats in the present disclosure.
FIG. 8 is a column chart showing the effect of different compounds on ESV of heart failure rats in the present disclosure.
FIG. 9 is a column chart showing the effect of different compounds on EDV of heart failure rats in the present disclosure.
FIG. 10 is a column chart showing the effect of different compounds on LVPWTs of heart failure rats in the present disclosure.
FIG. 11 is a column chart showing the effect of different compounds on LV mass of heart failure rats in the present disclosure.
FIG. 12 is a column chart showing the effect of different compounds on heart rate of heart failure rats in the present disclosure.
FIG. 13 is a column chart showing the effect of different compounds on arterial blood pressure (SBP) of heart failure rats in the present disclosure.
FIG. 14 is a column chart showing the effect of different compounds on arterial blood pressure (DBP) of heart failure rats in the present disclosure.
FIG. 15 is a column chart showing the effect of different compounds on arterial blood pressure (MBP) of heart failure rats in the present disclosure.
FIG. 16 is a column chart showing the effect of different compounds on heart failure rats and LVSP in the present disclosure.

### DETAILED DESCRIPTION

The present disclosure discloses use of an ester group-containing aromatic propionamide compound for the manufacturing of a medicament for the treatment of heart failure, which is verified by specific experiments below. EG017 as mentioned herein is C₂₅H₁₇F₃N₄O₄, and reference can be made to the content of patent document CN201410033958.0 for the preparation method and the structural formula of the EG017. In all the drawings of the present disclosure, the columns represent Sham, Model and EG017 groups, respectively, from left to right.

### Instruments used in experiments of the present disclosure

Small animal ventilator, model: V-200046; manufacturer: Harvard Apparatus.

Vevo small animal ultrasound imaging system, model: 1100; manufacturer: Visualsonics.

Powerlab 8/35 signal acquisition system, model: 8/35; manufacturer: ADinstrunents.

Animal experiment running platform, model: DB030; manufacturer: Beijing Zhishu Biological Technology Co., Ltd.

Analytical balance, model: SQP; manufacturer: sartorius.

Electronic balance, model: FA-2204; manufacturer: Bangxi Instrument Technology (Shanghai) Co., Ltd.

Electronic balance, model: MP5002; manufacturer: Changzhou Tianzhiping Instruments Co., Ltd.

Centrifuge, model: 5424 R; manufacturer: Eppendorf.

Constant temperature magnetic stirrer, model: 85-2; manufacturer: Shanghai Sile Instrument Co., Ltd.

Miniature vortex mixer, model: XW-80A; manufacturer: Shanghai Huxi Analysis Instrument Factory Co., Ltd.

The test compound used in the experiments of the present disclosure is shown below:

| **Test compound** | **Lot No.** | **Supplier** | **Storage conditions** |
|---|---|---|---|
| EG017 | Q18-078 | 2Y-Chem, Ltd. | Sealed, and stored at room temperature |

### Reagents:

CMC Na, manufacturer: Sigma; Lot#: SLBV9664; storage condition: room temperature.

Isoflurane, manufacturer: Jiangsu HFQ Bio-Technology Co., Ltd.; Lot#: 20191223; storage condition: sealed at room temperature.

Pentobarbital sodium injection, manufacturer: AlfaMedic Ltd; Lot#: 1709296-02; physical state: pink liquid, 0.2 g/mL; expiration date: September 2020; storage condition: sealed at room temperature.

Meloxicam injection, manufacturer: Qilu Animal Health Products Co., Ltd.; Lot#: 1710002; storage condition: sealed at room temperature.

Gentamicin sulfate injection, manufacturer: Huazhong Pharmaceutical Co., Ltd.; Lot#: 20180622; storage condition: in dark at room temperature.

Saline, manufacturer: Cisen Pharmaceutical Co., Ltd.; Lot#: 18101907; storage condition: sealed at room temperature.

### Vehicle and compound formulation

1. Vehicle (0.5% CMC Na): formulation: 5 g of CMC Na was weighed out, and the volume was made up to 1000 mL with ddwater; the vehicle was obtained by stirring with a stirrer until the CMC Na was dissolved.
2. EG017: formulation: a proper amount of the sample was weighed out, and a formula amount of the 0.5% CMC Na was added to prepare 1 mg/kg, 3 mg/kg and 10 mg/kg compound; the formulations were obtained by stirring with a stirrer until the sample was dissolved. **Administration regimen:** one week after animal modeling, sham/myocardial infarction rats were grouped and administered orally once daily for 28 consecutive days in a volume of 10 mL/kg.

### Use of experimental animals

Sprague Dawley rats (SD rats), supplied by Vital River Laboratory Animal Technology Co., Ltd.; female, 120 rats, license No.: SCXK (Jing) 2016-0006, experimental animal certificate No.: 1100112011006510. Female, 50 rats, license No.: SCXK (Zhe) 2019-0001, experimental animal certificate No.: 2003050021.

### Feeding of animals

The animals used in the following experiments were all SD rats. After the animals arrived at the facility of Shanghai-based WuXi AppTec, they were raised in an animal feeding room with strictly controlled environmental conditions (temperature maintained at 20-24 °C and the humidity maintained at 30-70%). The temperature and humidity of the feeding room were monitored in real time by a hygrothermograph and recorded twice daily (once in the morning and once in the afternoon). The lighting of the animal feeding room was controlled by an electronic timed light-on system, and the light was on for 12 hours a day and off for 12 hours a day (turned on at 6:00 AM and turned off at 18:00 PM). The animals had free access to food and water.

### Specific experimental method

After adaptive feeding, on the day of the experiment, the animals were anesthetized by intraperitoneal injection of pentobarbital sodium injection (60 mpk), atropine (0.5 mpk) was injected intraperitoneally to eliminate phlegm, and then the trachea was connected with a breathing machine to assist breathing; the chest was cut open from the place between the third rib and the fourth rib and then expanded with a chest expander; the pericardium was torn open, the left anterior descending coronary artery was ligated with 5-0 silk thread, the ribs and skin were sutured, and then the animals were placed on a heat preservation blanket for recovery after operation. The animals in the Sham group were also subjected to the same operation, except that the ligation with silk thread was not performed. After the operation was finished, all the animals are subjected to intramuscular injection of meloxicam (1 mpk) and gentamicin hydrochloride injection (8 mpk) for analgesia and anti-infection. One day before administration, the rats were subjected to cardiac ultrasonography after anesthesia by isoflurane inhalation (1.5%-5% v/v in oxygen), and the ligation was successful if there was no significant contraction of the anterior wall of the left ventricle, the left ventricular cavity was enlarged, and there was a 30% decrease in left ventricular ejection fraction compared to the normal control group. Successfully modeled rats were selected for subsequent experiments (note: ensure that there were 7 animals in each group after modeling, and 7 animals in the sham group). The rats in each group were subjected to intragastric administration of therapeutic agent once daily for four consecutive weeks. During the experiment, the living state of the animals was observed, and the abnormal conditions were recorded; endpoint index detection and sample collection were performed one day after the last dose.

One week after the modeling of animals, the isoflurane was used for anesthetizing the rats, the Vevo small animal ultrasonic imaging system was used for checking the function of the left ventricle of the model rats, and successful modeling was determined if the LVFE% was reduced by 30%.

Except for the Sham group, the animals were divided into 9 groups according to LVEF% and body weight, 7 rats in each group. The situation of each group is shown in the table below:

| **Grouping** | **Number of animals** | **Administration dose** | **Route of administration** | **Administration volume** | **Time of administration** |
|---|---|---|---|---|---|
| Sham | 7 | Vehicle | p.o, qd | 10 mL/kg | Beginning one week after modeling and continuing for four weeks. |
| Model | 7 | Vehicle | p.o, qd | 10 mL/kg | |
| EG017 | 7 | 3mg/kg | p.o, qd | 10 mL/kg | |

### Endpoint detection indexes

**A. Echocardiography** (one day after last dose): the animals were anesthetized by isoflurane inhalation (1.5%-5% v/v in oxygen) and then examined for left ventricular ejection fraction (LVEF), fractional shortening (FS), left ventricular end-systolic volume (ESV), left ventricular end-diastolic volume (EDV), stroke volume (SV), cardiac output (CO), left ventricular mass (LV mass), left ventricular end-systolic posterior wall thickness (LVPWTs) and left ventricular end diastolic posterior wall thickness (LVPWTd).
**B. Hemodynamics** (one day after the last dose): the rats were each anesthetized by using pentobarbital sodium (~60 mpk, ip) and fixed on an operating table in a supine position; the common carotid artery was separated, a catheter was inserted to detect the common carotid artery blood pressure (SP/DP) of the rat by a Powerlab system, and then the catheter was pushed into the left ventricle to detect the systolic pressure/diastolic pressure (LVSP/LVDP) of the left ventricle; the maximum rate of rise/fall (±dp/dtmax) of left ventricular pressure and heart rate (HR) were measured, and all the detection was finished within 30 min after anesthesia.
**C. Organ weight index:** heart weight/100 g body weight; heart/brain ratio: heart weight/brain weight.
**D. Myocardial blush:** Sirius red staining was performed on cardiac muscle, and myocardial fibrosis levels were observed.

### Data statistics

Data were expressed as Mean±S.E.M, and Graphpad Prism 5.0 was used for statistical cartography. One-way ANOVA Dunntt's test and t-test were used, and P < 0.05 represented that the difference was statistically significant.

### Preliminary observations

Research showed that the body weight of the rats in the test group which were provided with EG017 increased significantly; the endpoint anatomy of heart showed that the hearts of the rats in the Sham group were ruddy and full, while the left ventricle of the hearts of the rats in the model group were shriveled, and the infarct areas were grayish white.

### Effect of compound on heart weight index of heart-failure rats

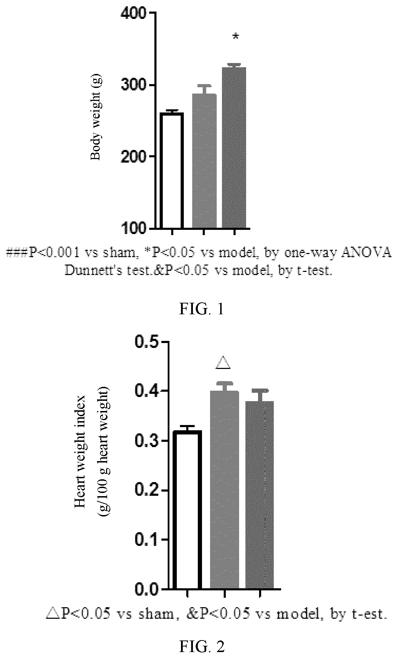

Endpoint data of rats were collected, and the rats were dissected. The heart was taken out, washed with normal saline, and weighed after absorbing water, and the heart weight index was calculated according to body weight. After the brain was taken out and weighed, the brain/heart weight ratio was calculated. As can be seen from FIGs. 2-3 and Table 1, the heart weight index and the brain/heart weight ratio of the Model group were significantly increased compared with those of the Sham group, and the heart weight index and the brain/heart weight ratio of the test group were not statistically significantly different from those of the Model group (P > 0.05)

**Table 1. Heart weight index and brain/heart weight ratio (Mean±SEM) of rats, N =7**

| **Group** | **Dose** | **Brain weight/heart weight (g/g)** | **Heart weight index (g/100 g body weight)** |
|---|---|---|---|
| Sham | Vehicle | 58.0 ± 1.91 | 0.318 ± 0.012 |
| Model | Vehicle | 76.9 ± 3.99# | 0.398 ± 0.018Δ |
| EG017 | 3mpk | 78.3 ± 4.31 | 0.376 ± 0.025 |

| | | | |
|---|---|---|---|
| △ P < 0.05 vs sham, & P < 0.05 vs model, by t-test; # P < 0.05 vs sham, by one-way ANOVA Dunnett's test. | | | |

### Effect of compound on cardiac function of rats

### Effect of compound on cardiac contractile function of rats

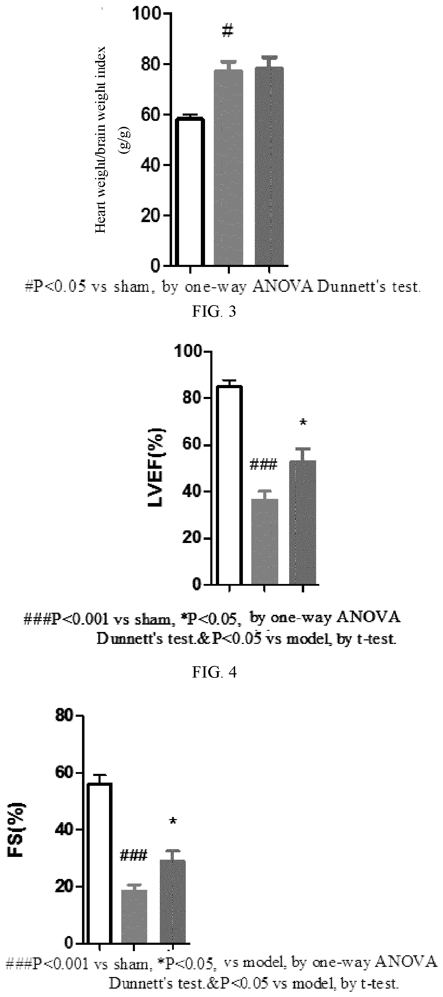

The heart failure was mainly characterized by a decreased contractile function of the left ventricle, and therefore at the end of the experiment, the hearts of the rats in all the groups were examined by longitudinal echocardiography, and the changes of left ventricular ejection fraction (LVEF), fractional shortening (FS), cardiac output (CO) and stroke volume (SV) were analyzed and compared. From FIGs. 4-7 and Table 2, it can be seen that the rats in the Model group developed severe left ventricular dysfunction after myocardial infarction, and the LVEF, FS, CO and SV were 36.3±3.83%, 18.5±2.22%, 55.7±4.90 mL/min and 159±8.62 µL, respectively, which were significantly lower than 84.8±2.83%, 56.1±3.14%, 77.2±5.10 mL/min and 187±9.46 µL of the Sham group, showing statistically significant differences (P < 0.001, P < 0.001, P < 0.05 and P < 0.05). Compared with the Model group, the EG017 group showed significant improvement effect on the reduction of LVEF and FS of heart failure rats, and the statistic difference was significant (P < 0.05). The EG017 group showed significant improvement effect on the reduction of CO and SV (P < 0.05).

**Table 2. Effect of compound on LVEF, FS, CO and SV of heart failure rats (Mean±SEM), N = 7**

| **Group** | **Dose** | **LVEF (%)** | **FS (%)** | **CO(mL/min)** | **SV(µL)** |
|---|---|---|---|---|---|
| Sham | Vehicle | 84.8±2.83 | 56.1±3.14 | 77.2±5.10 | 187±9.46 |
| Model | Vehicle | 36.3±3.83### | 18.5±2.22### | 55.7±4.90△ | 159±8.62△ |
| EG017 | 3mpk | 52.7±5.61* | 29.0±3.51* | 85.6±9.55** | 238±21.1** |

| | | | | | |
|---|---|---|---|---|---|
| ***# < 0.001 vs sham, * P < 0.05, ** P < 0.01 vs Model, by one-way ANOVA Dunnett's test; △ P < 0.05 vs sham, & P < 0.05, && P < 0.01 vs Model by t-test. | | | | | |

### Effect of compound on left ventricular volume of rats

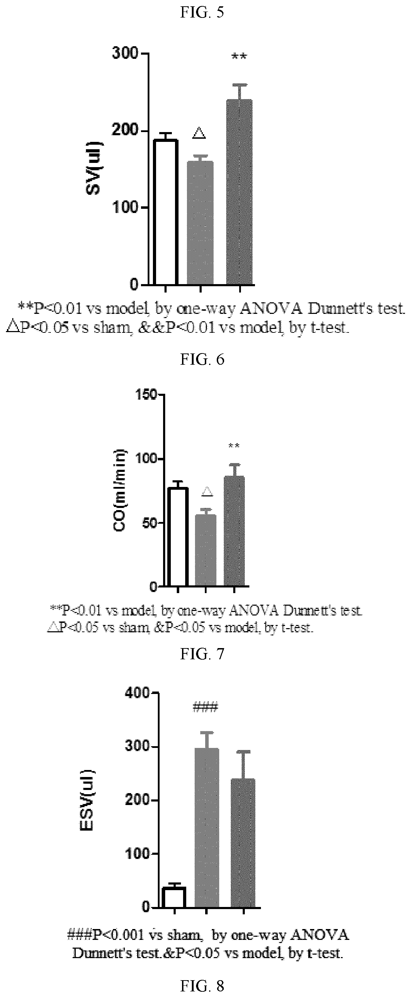

After heart failure develops, the left ventricular cavity becomes larger and the posterior wall becomes thinner due to compensation. As can be seen from FIGs. 8-9 and Table 3, compared with the Sham group, the heart failure rats showed significant increase in both cardiac EDV and ESV, and the statistic difference was extremely significant (P < 0.001). Compared with the Model group, the EG017 group 8 showed significant improvement effect in the increase of systolic volume (ESV) of the heart due to heart failure (P < 0.05).

**Table 3. Effect of compound on left ventricular systolic volume and diastolic volume of heart failure rats (Mean±SEM), N = 7**

| **Group** | **dose** | **ESV (µL)** | **EDV (µL)** |
|---|---|---|---|
| Sham | Vehicle | 36.0±9.05 | 223±16.1 |
| Model | Vehicle | 294±32.6### | 453±28.5### |
| EG017 | 3mpk | 238±52.0 | 477±48.2 |

| | | | |
|---|---|---|---|
| ### P < 0.001 vs Sham, by one-way ANOVA Dunnett's test; & P < 0.05 vs model, by t-test. | | | |

### Effect of compound on ventricular posterior wall thickness and heart mass of rats

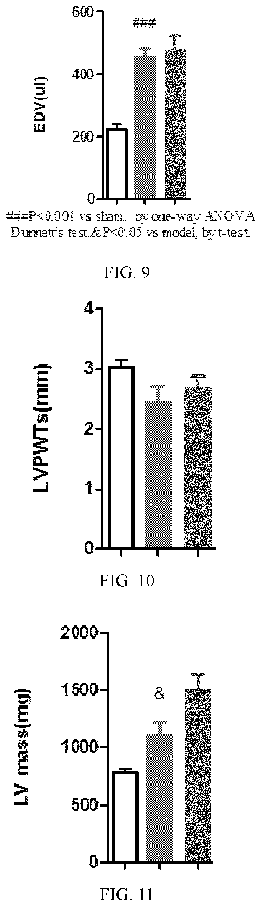

As can be seen from FIGs. 10-11 and Table 4, compared with the Sham group, the rats in the Model group showed slightly decreased LVPWTs and LVPWTd (P > 0.05) but significantly increased heart mass (P < 0.05), and the statistic difference was significant. Compared with the Model group, the effect of each administration group on LVPWTs, LVPWTd and LV mass was not significant (P > 0.05).

**Table 4. Effect of compound on left ventricular posterior wall thickness and left ventricular mass of heart failure rats (Mean±SEM), N = 7**

| **Group** | **dose** | **LVPWTs (mm)** | **LVPWTd (mm)** | **LV mass (mg)** |
|---|---|---|---|---|
| Sham | Vehicle | 3.03±0.12 | 1.75±0.06 | 780±30.4 |
| Model | Vehicle | 2.44±0.27 | 1.81±0.17 | 1107±113& |
| EG017 | 3mpk | 2.66±0.22 | 2.17±0.17 | 1497±145 |

| | | | | |
|---|---|---|---|---|
| & P < 0.05 vs model by t-test. | | | | |

### Effect of compound on hemodynamics of heart failure rats

After myocardial infarction, the left ventricle of the rat was subjected to myocardial infarction, which manifested the contractile function of the left ventricle to be reduced, with the manifestations of the reduction of ventricular systolic pressure and the reduction of the rising/falling speed (±dp/dt max) of the ventricular pressure.

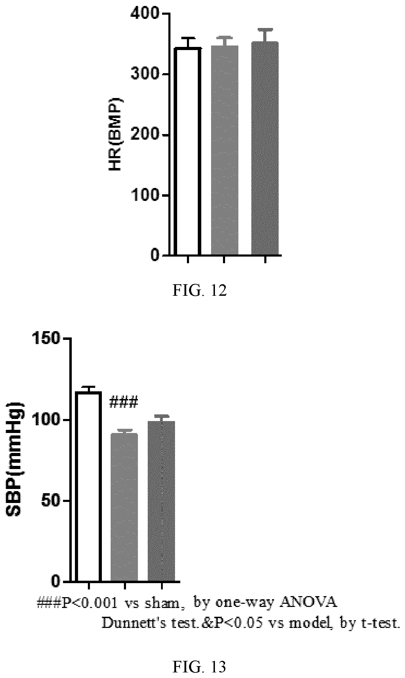

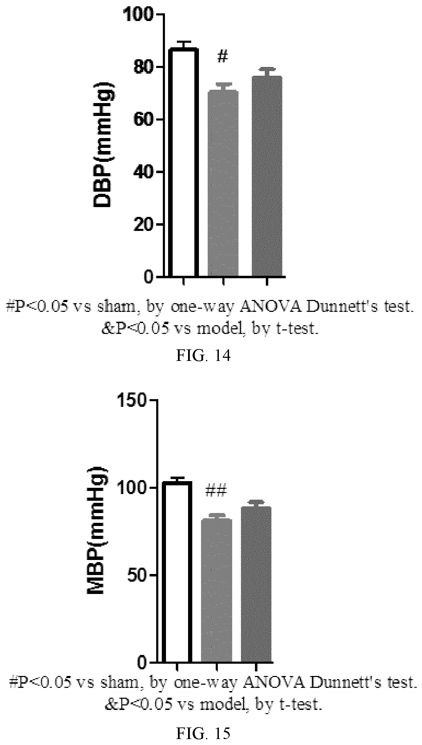

As can be seen from FIGs. 12-15 and Table 5, the heart rate was not significantly affected (P > 0.05) in all the experimental groups. The arterial blood pressures (SBP, DBP and MBP) of the Model group were significantly reduced compared with those of the Sham group, and the statistic differences were significant (P < 0.001, P < 0.05, and P < 0.01). Compared with the Model group, the EG017 group showed no significant influence on the arterial blood pressures of rats after heart failure.

**Table 5. Effect of compound on heart rate and arterial blood pressures of heart failure rats (Mean±SEM), N = 7**

| **Group** | **dose** | **HR (BPM)** | **SBP (mmHg)** | **DBP (mmHg)** | **MBP (mmHg)** |
|---|---|---|---|---|---|
| Sham | Vehicle | 343±17.4 | 117±3.33 | 86.6±2.95 | 103±3.09 |
| Model | Vehicle | 345±15.3 | 90.7±3.14### | 70.3±3.26# | 81.2±3.09## |
| EG017 | 3mpk | 352±23.3 | 98.7±3.62 | 76.1±3.02 | 88.3±3.39 |

| | | | | | |
|---|---|---|---|---|---|
| # P < 0.05, ## P < 0.01, ### P < 0.001 vs sham, * P < 0.05 vs model, by one-way ANOVA Dunnett's test; & P < 0.05 vs model, by t-test. | | | | | |

### Effect of compound on ventricular pressure and rate of ventricular pressure change

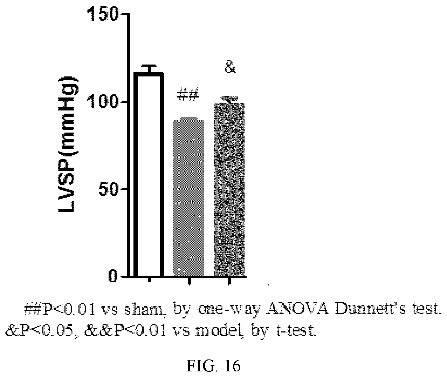

Ventricular pressures are as shown in FIG. 16 and Table 6. Compared with the Sham group, the rats in the Model group showed significant reduction in left ventricular systolic pressure (LVSP), and the statistic difference was significant (P < 0.01). Compared with the Model group, EG017 showed relatively significant effect on LVSP reduction caused by heart failure, and the statistic difference was significant (P < 0.05).

Compared with the Sham group, the +dp/dt max and the -dp/dt max of the rats in the Model group, which were 5164 ± 352 mmHg/s and -3789 ± 220 mmHg/s, respectively, were significantly lower than 6210 ± 429 mmHg/s and 5091 ± 456 mmHg/s, respectively, of the Sham group, and the statistic differences were significant (P < 0.05 and P < 0.01).

**Table 6. Effect of compound on left ventricular blood pressure of heart failure rats (Mean±SEM), N = 7**

| **Group** | **dose** | **LVSP (mmHg)** | **Dp/dt max (mmHg)** | **Dp/dt min (mmHg)** |
|---|---|---|---|---|
| Sham | Vehicle | 116±4.87 | 9223±574 | -9052±890 |
| Model | Vehicle | 88.4±1.75## | 5678±290### | -5124±498## |
| EG017 | 3mpk | 98.5±3.89& | 6630±432 | -6603±707 |

| | | | | |
|---|---|---|---|---|
| ## P < 0.01, ### P < 0.001 vs sham, * P < 0.05 vs model, by one-way ANOVA Dunnett's test; & P < 0.05, && P < 0.01 vs model, by t-test. | | | | |

The following example was used to demonstrate that the metabolite of EG017 has a positive effect in preparing a medicament for the treatment of heart failure.

### 2.1. Test sample and vehicle

### 2.1.1. Test sample

Name: EG017; physical state: off-white powder;
Main component: (*S*)-1-((4-cyano-3-(trifluoromethyl)phenyl)amino)-3-(4-cyanophenoxy)-2-methyl-1-oxopropan-2-yl nicotinate; content: 99.3%;

### 2.1.2. Vehicle

### 2.1.2.1. CMC-Na

Name/code number: sodium carboxymethylcellulose/CMC-Na; appearance: white or yellowish fibrous powder;

### 2.1.2.2. DMA

Name/code number: *N,N*-dimethylacetamide/DMA;
appearance: colorless or near-colorless clear liquid;

### 2.1.2.3. solutol

Name/code number: polyethylene glycol (15)-hydroxystearate/solutol; appearance: yellowish paste at room temperature and liquid at about 30 °C, dissolved in water or ethanol to form a clear solution;
Storage condition: 2-8 °C;

### 2.1.2.4. Sodium chloride injection

Name: sodium chloride injection;
Lot#: 1804172726; appearance: colorless transparent liquid;

### Preparation of test sample

### Test sample suspension

A certain amount of CMC-Na (±1% weighing error allowed) was weighed out and dissolved in purified water, thus preparing 0.5% CMC-Na. Preparation method of test sample suspension: a certain amount of EG017 was weighed out according to the following table, placed in a mortar, fully ground by adding a small amount of 0.5% CMC-Na and then transferred into a container; the mortar was washed 4 times, the washing liquid was transferred into the container, and 0.5% CMC-Na was added for diluting to a required volume, thus preparing the test sample suspension with a required concentration. The suspension was stirred with a magnetic stirrer at a rotation speed of 1200 rpm for at least 15 min prior to sampling for analysis and drug administration.

In the experiment, 32 beagle dogs were used and randomly divided into 4 groups (8 dogs for each, half male and half female), which were set as an intravenous injection group (1 mg/kg) and a dynamics low-dose group (1 mg/kg), a dynamics medium-dose group (3 mg/kg) and a dynamics high-dose group (10 mg/kg) of intragastric administration. Blood samples were collected at corresponding time points after administration and subjected to EDTA-K2 anticoagulation. The animals in the medium-dose group were subjected to intragastric administration for 7 consecutive days after blood sample collection at the corresponding time points. The concentration of EG017 and the main metabolite EG-2 in plasma was detected using the LC-MS/MS method, and pharmacokinetic parameters were calculated by using the WinNonlin 6.4 software.

## Claims

1. An ester group-containing aromatic propionamide compound for use in the treatment of heart failure, wherein the ester group-containing aromatic propionamide compound is C₂₅H₁₇F₃N₄O₄ and the chemical name thereof is (*S*)-1-((4-cyano-3-(trifluoromethyl)phenyl)amino)-3-(4-cyanophenoxy)-2-methyl-1-oxopropan-2-yl nicotinate.

2. The compound for use of claim 1, wherein a dose of the ester group-containing aromatic propionamide compound used in the treatment of heart failure is 1 mg/kg-10 mg/kg.

3. The compound for use of claim 1, wherein the dose of the ester group-containing aromatic propionamide compound used in the treatment of heart failure is 3 mg/kg.

## Patentansprüche

1. Estergruppen enthaltende aromatische Propionamidverbindung zur Verwendung bei der Behandlung von Herzinsuffizienz, wobei die Estergruppen enthaltende aromatische Propionamidverbindung C₂₅H₁₇F₃N₄O₄ ist und der chemische Name davon (S)-1-((4-Cyano-3-(trifluormethyl)phenyl)amino)-3-(4-cyanophenoxy)-2-methyl-1-oxopropan-2-yl-nicotinat ist.

2. Verbindung zur Verwendung nach Anspruch 1, wobei eine Dosis der Estergruppen enthaltenden aromatischen Propionamidverbindung, die zur Behandlung von Herzinsuffizienz verwendet wird, 1 mg/kg bis 10 mg/kg beträgt.

3. Verbindung zur Verwendung nach Anspruch 1, wobei die Dosis der Estergruppen enthaltenden aromatischen Propionamidverbindung, die zur Behandlung von Herzinsuffizienz verwendet wird, 3 mg/kg beträgt.

## Revendications

1. Composé de propionamide aromatique contenant un groupe ester pour une utilisation dans le traitement de l'insuffisance cardiaque, dans lequel le composé de propionamide aromatique contenant un groupe ester est en C₂₅H₁₇F₃N₄O₄ et le nom chimique de celui-ci est nicotinate de (S)-1-((4-cyano-3-(trifluorométhyl)phényl)amino)-3-(4-cyanophénoxy)-2-méthyl-1-oxopropan-2-yle.

2. Composé pour une utilisation selon la revendication 1, dans lequel une dose du composé de propionamide aromatique contenant un groupe ester utilisé dans le traitement de l'insuffisance cardiaque est de 1 mg/kg à 10 mg/kg.

3. Composé pour une utilisation selon la revendication 1, dans lequel la dose du composé de propionamide aromatique contenant un groupe ester utilisé dans le traitement de l'insuffisance cardiaque est de 3 mg/kg.
